(19) Europäisches Patentamt
European Patent Office
Office européen des brevets

(11) Veröffentlichungsnummer: **0 003 570**
**B1**

(12) **EUROPÄISCHE PATENTSCHRIFT**

(21) Anmeldenummer: **79100302.3**

(22) Anmeldetag: **02.02.79**

(51) Int. Cl.³: **C 07 C 118/02,**
C 07 C 119/042

(54) **Verfahren zur kontinuierlichen Aufarbeitung von bei der Phosgenierung von Monoaminen anfallenden Lösungen**

(30) Priorität: **14.02.78 DE 2806214**

(43) Veröffentlichungstag der Anmeldung:
**22.08.79 Patentblatt 79/17**

(45) Bekanntmachung des Hinweises auf die Patenterteilung:
**23.07.80 Patentblatt 80/15**

(84) Benannte Vertragsstaaten:
**BE DE FR GB IT NL**

(56) Entgegenhaltungen:
**DE - A - 2 411 441**
**DE - A - 2 800 153**

(73) Patentinhaber: **Bayer AG**
**Zentralbereich Patente, Marken und Lizenzen**
**D - 5090 Leverkusen 1, Bayerwerk (DE)**

(72) Erfinder: **Koster, Johannes, Dr.**
**Schulstrasse 46**
**D - 4047 Dormagen 11 (DE)**
**Heitkämper, Peter, Dr.**
**Wiedstrasse 6**
**D - 4047 Dormagen (DE)**
**Fuhrmann, Peter**
**Stürzelbergerstrasse 77**
**D - 4047 Dormagen 5 (DE)**
**Porkert, Helmut, Dr.**
**Moselstrasse 11**
**D - 4047 Dormagen (DE)**

**EP 0 003 570 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur kontinuierlichen Aufarbeitung von bei der Phosgenierung von Monoaminen anfallenden Lösungen

Die Erfindung betrifft ein verbessertes Verfahren zur kontinuierlichen Herstellung von Alkylmonoisocyanaten aus technischen, N-Alkylcarbamidsäurechloride enthaltenden Lösungen durch thermische Zersetzung der N-Alkylcarbamidsäurechloride in Gegenwart von inerten Lösungsmitteln und destillativer Gewinnung der reinen Monoisocyanate.

Isocyanate werden bekannterweise durch Umsetzung von Aminen mit Phosgen hergestellt. Die Reaktion verläuft über die Stufe der Carbamidsäurechloride, die bei höherer Temperatur in die entsprechenden Isocyanate und Chlorwasserstoff spalten. Liegt der Siedepunkt des herzustellenden Isocyanats deutlich über der Zersetzungstemperatur des Carbamidsäurechlorids, so kann der bei der Zersetzung gebildete Chlorwasserstoff, insbesondere bei Verwendung eines inerten organischen Lösungsmittels, problemlos aus dem Reaktionsraum entfernt werden. Wenn jedoch die Zersetzungstemperatur des Carbamidsäurechlorids in der Nähe oder oberhalb des Siedepunkts des Isocyanats liegt, so gelangt das Isocyanat in den Gasraum, wo es mit dem Chlorwasserstoff zu Carbamidsäurechlorid rekombiniert. Deshalb verläuft die Spaltung unvollkommen und das Isocyanat wird mit Carbamidsäurechlorid verunreinigt erhalten.

Der geschilderte Sachverhalt trifft für aliphatische Monoisocyanate zu, deren aliphatische Reste 1 bis 3 Kohlenstoffatome enthalten, wobei die Schwierigkeiten bei der Herstellung von Methylisocyanat am größten sind.

In der Patentliteratur sind zahlreiche Verfahren beschrieben, die die genannten Schwierigkeiten beseitigen sollen. Ein Großteil der Veröffentlichungen beschreibt die Spaltung von Carbamidsäurechloriden unter Verwendung von Chlorwasserstoffakzeptoren.

So ist bekannt, Isocyanate aus Carbamidsäurechloriden in Gegenwart von organischen Basen (z.B. tertiären Aminen) oder Carbonsäure-dialkylamiden (DE—OS 1 593 554) oder Tetraalkylharnstoffen (US—PS 3 644 461) in organischen Lösungsmittel herzustellen. Ferner wird zur Absorption des Chlorwasserstoffs die Verwendung von Wasser (DE—AS 2 156 761) und von wäßrigen Lösungen oder Suspensionen anorganischer Basen (GB—PS 1 208 862) beschrieben. Auch Olefine werden als Chlorwasserstoffakzeptoren genannt (DE—OS 2 210 285).

Alle genannten Verfahren weisen den gravierenden Nachteil auf, daß Nebenprodukte entstehen (korrosive organische oder anorganische Salze oder Alkylchloride), die entweder kostspielig aufgearbeitet werden müssen oder aber eine Umweltbeeinträchtigung darstellen. Darüber hinaus besteht bei Verwendung von organischen Basen die Gefahr von Nebenreaktionen zu di- und trimeren Isocyanaten. In Gegenwart von Wasser wird ein beträchtlicher Teil des Carbamidsäurechlorids zum Amin-hydrochlorid hydrolysiert.

Es ist auch bekannt, aus den N-Alkylcarbamidsäurechloriden zunächst durch Umsetzung mit aliphatischen oder aromatischen Hydroxyl-Verbindungen unter Abspaltung und Abtrennung von Chlorwasserstoff die entsprechenden N-Alkylcarbamidsäureester herzustellen und dann durch thermische Spaltung dieser Carbamidsäureester die Isocyanate zu gewinnen. (Houben-Weyl, Methoden der organischen Chemie, Band 8, Seite 126, 1952).

So lassen sich gemäß der US—PS 3 076 007 aus N-Alkylcarbamidsäure-2-hydroxyäthylestern und gemäß der DE—OS 2 512 514 aus N-Alkylcarbamidsäure-$\beta$-naphthylestern durch thermische Spaltung die entsprechenden Monoisocyanate freisetzen.

Die Nachteile dieser aufwendigen, mehrstufigen Verfahren bestehen darin, daß einerseits die Spaltprodukte im Anschluß an die thermische Spaltung teilweise wieder zu den Carbamidsäureestern rekombinieren und andererseits sich in den hochsiedenden Hydroxyl-Verbindungen nicht-flüchtige Nebenprodukte anreichern.

Ferner ist auch bereits die direkte Herstellung von niedrigsiedenden aliphatischen Monoisocyanaten durch thermische Spaltung von Carbamidsäurechloriden in organischen Lösungsmitteln unter Anwendung spezieller Verfahrenstechniken bekannt.

Nach DT—AS 1 193 034 bzw. US—PS 3 388 145 wird die thermische Spaltung des Carbamidsäurechlorids in einem mit Rückflußkühler und trennwirksamer Kolonne versehenen Reaktor durchgeführt. Durch den Rückflußkühler entweicht Chlorwasserstoff, während Isocyanat, Carbamidsäurechlorid und Lösungsmittel zurückgehalten werden. Das gebildete Isocyanat gelangt in die Kolonne und kann am Kolonnenkopf abgenommen werden. Durch einen Rücklaufteiler wird der größte Teil des Isocyanats zurückgeführt, damit in die Kolonne aufsteigendes Carbamidsäurechlorid in den Reaktor zurückgelangt.

Obwohl dieses Verfahren zur diskontinuierlichen Herstellung von Alkylisocyanaten insbesondere im Labormaßstab gut geeignet ist, weist es noch schwerwiegende Nachteile auf, die ein kontinuierliches Arbeiten in großtechnischem Maßstab nach dem Prinzip dieser Veröffentlichungen erheblich erschweren. Diese Nachteile sind:

1. Von dem im Reaktor und in der Kolonne vorhandenen Isocyanat kann nur ein kleiner Teil am Kopf der Kolonne entnommen werden, während ein großer Teil dieses Isocyanats zusammen mit dem restlichen nicht gespaltenen Carbamidsäurechlorid als Sumpflösung aus dem Reaktor entnommen wird. Wird dennoch mehr Produkt an Kolonnenkopf entnommen, so stellt dieses entnommene Produkt kein reines Monoisocyanat dar. Vielmehr können dann lediglich Gemische mit wesentlichen Anteilen an Carbamidsäurechlorid am Kolonnenkopf gewonnen werden. Dieser Nachteil kann auch nicht durch trennwirksamere Kolonnen ausgeglichen werden.

2. Zur Gewinnung von Carbamidsäurechloridfreiem Isocyanat am Kolonnenkopf muß die Kolonne mit hohen Rücklaufverhältnissen, also mit hohem Energieaufwand betrieben werden.
3. Zur Spaltung des restlichen, nicht gespaltenen Carbamidsäurechlorids muß die aus dem Sumpf des Reaktors entnommene, Isocyanat-haltige Carbamidsäurechlorid-Lösung, gegebenenfalls nach Abtrennung eines Teils des Lösungsmittels, wieder in den Reaktor zurückgeführt werden. Damit ergibt sich zwangsläufig ein entsprechend großer Isocyanat-Kreislauf, der letztlich sehr geringe Raum-Zeit-Ausbeuten zur Folge hat.
4. Dieser große Isocyanat-Kreislauf führt weiterhin dazu, daß sich innerhalb des Reaktors hohe Isocyanat-Konzentrationen einstellen. Die leicht flüchtigen Isocyanate werden dann beim Erhitzen der Lösungen bevorzugt verdampft, so daß die zum Rückflußkühler aufsteigenden Produkt-Dämpfe entsprechend hohe Isocyanat-Gehalte besitzen. Das hat schließlich zur Folge, daß die Effektivität der thermischen Chlorwasserstoff-Abspaltung am Rückflußkühler und damit die Raum-Zeit-Ausbeute drastisch vermindert werden.

Diese Nachteile führen dazu, daß bei dem genannten Verfahren bei kontinuierlicher Fahrweise die Reaktionsprodukte, sowie das zur Spaltung der Carbamidsäurechloride notwendige Lösungsmittel mehrfach im Kreis geführt werden müssen. Durch das wiederholte Verdampfen und Kondensieren der Gemische und durch die notwendigen hohen Rücklaufverhältnisse in der Destillationskolonne des Reaktors ergeben sich einerseits ein hoher Energieverbrauch, andererseits gravierende Ausbeuteverluste durch Bildung von höhermolekularen Folgeund Nebenprodukten aus dem Isocyanat bzw. Carbamidsäurechlorid (vgl. z.B. H. Ulrich et al, J. Org. Chem. *29*, 2401 (1964)).

In den deutschen Offenlegungsschriften 2 411 441, 2 411 442, 2 422 211 und 2 503 270 werden Verfahrensvarianten beschrieben, die ebenfalls auf dem im vorgenannten Verfahren angegebenen Prinzip beruhen.

So wird in der DE—OS 2 411 441 (≙ US—PS 3 969 389) ein Verfahren beansprucht, in dem in einem Reaktor mit Rückflußkühler zuerst das Carbamidsäurechlorid durch Erhitzen der Carbamidsäurechlorid-Lösung am Rückfluß teilweise in Isocyanat und Chlorwasserstoff gespalten wird, dann aber die Isolierung des Isocyanats in einem separat angeordneten Apparat durchgeführt wird.

Bei diesem Verfahren haben die oben angegebenen Nachteile ebenfalls Gültigkeit; darüber hinaus ist der apparative Aufwand groß.

In der DE—OS 2 411 442 (≙ US—PS 3 991 094) ist ein Verfahren beschrieben, in dem während der thermischen Spaltung des Carbamidsäurechlorid am Rückfluß ein Inertgasstrom durch das Reaktionsgemisch geleitet wird, der den Chlorwasserstoff aus dem Reaktionsraum entfernen und so die Spaltung des Carbamidsäurechlorids fördern oder sogar praktisch vollständig ablaufen lassen soll. Tatsächlich wird aber mit diesem Verfahren keine nachweisliche Steigerung der Carbamidsäurechlorid-Spaltung erreicht, da der Inertgasstrom keine selektive rektifizierende Wirkung besitzt, so daß mit dem Inertgas nicht nur erhöhte Mengen Chlorwasserstoff, sondern auch entsprechend erhöhte Mengen an niedrigsiedendem Isocyanat aus dem Reaktionsraum entfernt werden. Außerdem erfordert das Verfahren einem großen apparativen Aufwand.

Die genannten Nachteile gelten auch für das Verfahren der DE—OS 2 503 270. In diesem Mehrstufen-Verfahren wird in der ersten Stufe eine Carbamidsäurechlorid-Lösung durch thermische Spaltung am Rückfluß behandelt und die dabei entstehende Lösung dann in der zweiten Stufe unter Durchleiten eines Inertgasstromes wiederum am Rückfluß erhitzt, wobei das restliche Carbamidsäurechlorid praktisch vollständig in Isocyanat übergeführt werden soll.

Schließlich wird in der DE—OS 2 422 211 (≙ US—PS 3 969 388) ein Verfahren beansprucht, in dem die Chlorwasserstoff-Abspaltung aus dem Carbamidsäurechlorid durch thermische Behandlung der Lösungen am Rückfluß in 2 bis 6 aufeinanderfolgenden Reaktionsräumen durchgeführt und anschließend das Isocyanat isoliert wird. Tatsächlich wird aber bei diesem Verfahren in dem zweiten Reaktionsraum und in den folgenden Reaktionsräumen keine nachweisliche Menge an Chlorwasserstoff abgespalten. Das könnte nur erreicht werden, wenn aus den Lösungen, die aus den Reaktionsräumen entnommen werden, zunächst Isocyanat isoliert wird und dann die Lösungen in den nachfolgenden Reaktionsraum eingegeben werden.

Aufgabe der vorliegenden Erfindung war es nun, ein Verfahren zur kontinuierlichen Aufarbeitung von Carbamidsäurechlorid enthaltenden, technischen Lösungen und Gewinnung des entsprechenden reinen Monoisocyanats in hoher Ausbeute zur Verfügung zu stellen, bei dem unerwünscht große Produktkreisläufe, hohe Produktverluste durch Bildung von Folge- und Nebenprodukten und ein unerwünscht hoher Energieaufwand vermieden werden.

Diese Aufgabe konnte durch das nachstehend näher erläuterte erfindungsgemäße Verfahren gelöst werden.

Gegenstand der vorliegenden Erfindung ist ein Verfahren zur kontinuierlichen Aufarbeitung von technischen Lösungen, wie sie bei der Phosgenierung von Monoaminen der Formel

$$R—NH_2$$

in welcher

R für einen gegebenenfalls ungesättigten Alkylrest mit 1 bis 3 Kohlenstoffatomen steht, anfallen, und welche im wesentlichen aus Carbamidsäurechloriden der Formel

$$R—NH—CO—Cl$$

gegebenenfalls Monoisocyanaten der Formel

$$R—NCO$$

wobei

R die obengenannte Bedeutung hat,

und inerten, oberhalb 80°C und mindestens 10°C über dem Siedepunkt des Isocyanats R—NCO siedenden Lösungsmitteln bestehen, unter Gewinnung des reinen Monoisocyanat R—NCO durch thermische Spaltung des entsprechenden Carbamidsäurechlorids und Destillation des dadurch gewonnenen und des gegebenenfalls bereits vorliegenden Isocyanats, dadurch gekennzeichnet, daß man die aufzuarbeitende Lösung an einer beliebigen Stelle in einen Produktkreislauf einspeist, der dadurch hergestellt worden ist, daß

a) man eine Lösung, welche im wesentlichen aus dem zu gewinnenden Monoisocyanat, dem zu spaltenden Carbamidsäurechlorid und dem genannten Lösungsmittel besteht, oberhalb des Kolonnensumpfes in eine aus einer oder mehreren Kolonnen bestehenden Destillationseinheit einspeist, an deren Kopf das reine Monoisocyanat und an deren Sumpf das gegebenenfalls noch verunreinigte Lösungsmittel anfallen, wobei im Falle der Verwendung einer aus mehreren Kolonnen bestehenden Destillationseinheit die Kolonnen so hintereinander geschaltet sind, daß ihre technische Funktion der Funktion einer einzigen Kolonne entspricht,

b) man der Destillationseinheit oberhalb der in a) genannten Einspeisstelle einen flüssigen Produktstrom entnimmt, der in wesentlichen aus einer konzentrierten, gegebenenfalls Monoisocyanat enthaltenden Carbamidsäurechlorid-Lösung in den genannter Lösungsmitteln besteht, und diesen Produktstrom mit zumindest einem Teil des gemäß a) erhaltenen Sumpfprodukts vereinigt,

c) man die gemäß b) erhaltenen, vereinigten Ströme in ein mit einen Rückflußkühler versehenes Reaktionsgefäß einbringt, oder die in b) genannte Vereinigung innerhalb des genannten Reaktionsgefäßes durchführt, wobei man durch Erhitzen der Ströme vor und/oder nach ihrer Vereinigung, bzw. vor und/oder nach dem Einbringen in das Reaktionsgefäß für ein zumindest teilweises Verdampfen der flüssigen Phase unter zumindest teilweiser Spaltung des Carbamidsäurechlorids in Monoisocyanat und Chlorwasserstoff Sorge trägt,

d) man den dabei entstehenden Chlorwasserstoff oberhalb des Rückflußkühlers gasförmig entweichen läßt und gleichzeitig zumindest einen Teil des sich am Rückflußkühler bildenden Kondensats als gemäß a) in die Destillationseinheit einzuspeisende Lösung an den Kreislaufanfang gemäß a) zurückführt, wobei man schließlich dem in c) genannten Reaktionsgefäß gleichzeitig kontinuierlich gegebenenfalls verunreinigtes Lösungsmittel als Flüssigkeit entnimmt.

Ausgangslösungen für das erfindungsgemäße Verfahren sind technische Lösungen, welche bei der Phosgenierung vom Monoaminen der Formel

$$R—NH_2$$

anfallen, in welcher

R die Bedeutung eines gegebenenfalls ungesättigten Alkylrestes mit 1 bis 3 Kohlenstoffatomen hat, wobei R insbesondere eine Methyl-, Äthyl-, n-Propyl-, iso-Propyloder Allylgruppe bedeutet, vorzugsweise aber für eine Methylgruppe steht.

Die genannten Ausgangslösungen stellen im wesentlichen Lösungen der genannten Carbamidsäurechloride der Formel

$$R—NH—CO—Cl$$

und gegebenenfalls der entsprechenden Monoisocyanate der Formel

$$R—NCO$$

in einem inerten Lösungsmittel, wie es als Reaktionsmedium bei der Phosgenierung bzw. im Falle einer Gasphasen-Phosgenierung zur Aufnahme der Reaktionsgase verwendet wird, und welches im allgemeinen einen oberhalb 80°C und mindestens 10°C über dem Siedepunkt des herzustellenden Monoisocyanats liegenden Siedepunkt aufweist, dar.

Beispiele geeigneter derartiger Lösungsmittel sind n-Heptan, n-Amylchlorid, 1,2-Dichlorpropan, isomere Dichlorbutane, Toluol, Xylole, Äthylbenzol, Chlorbenzol, Dichlorbenzol, Essigsäurebutylester, Buttersäureäthylester. Selbstverständlich können auch mehrere Lösungsmittel in den Ausgangslösungen enthalten sein. Vorzugsweise werden jedoch Ausgangslösungen verwendet, die als Lösungsmittel Chlorbenzol enthalten.

Die für das erfindungsgemäße Verfahren eingesetzten technischen Ausgangslösungen enthalten die genannten Carbamidsäurechloride in Konzentrationen von 0,5 bis 40, vorzugsweise 2 bis 15 Gew.-% und die gegebenenfalls enthaltenen genannten Monoisocyanate in Konzentrationen von 0 bis 30, vorzugsweise 0 bis 10 Gew-%.

Derartige technische Ausgangsverbindungen werden nach bekannten Methoden des Standes der Technik hergestellt. Sie können z.B. durch Phosgenierung von Lösungen der entsprechenden Monoamine in den genannten Lösungsmitteln erhalten werden (vgl. z.B. W. Siefken, Liebigs Ann. Chem. *562*, (1949)).

In sehr einfacher Weise werden diese Lösungen jedoch durch Phosgenierung der entsprechenden Monoamine in der Gasphase und anschließende Absorption der dabei entstehenden Reaktionsgase in den genannten Lösungsmitteln hergestellt.

Dabei wird das gasförmige Monoamin mit einer Temperatur von 0—300°C mit mindestens 1 Mol pro Mol Monoamin gasförmigem Phosgen mit einer temperatur von 10—300°C gegebenenfalls im Gemisch mit einem verdünnten Inertgas oder dampfförmigen Lösungsmittel umgesetzt. Die Reaktionstemperaturen liegen dabei im allgemeinen zwischen 240 und 400, vorzugsweise 300 bis 360°C.

Beispielhaft für eine derartige Umsetzung in der Gasphase sei die folgende Phosgenierung von Methylamin genannt:

In einen handelsüblichen zylindrischen statischen Mischer der Firma KENICS werden kontinuierlich 150 Mol pro Stunde auf 180°C erhitztes Phosgen-Gas eingeleitet. Gleichzeitig werden kontinuierlich direkt vor dem statischen Mischer 100 Mol pro Stunde auf 180°C erhitztes Methylamin-Gas koaxial in den Phosgen-Gasstrom eingeleitet. Dabei sind die Abmessungen des statischen Mischers so gewählt, daß die Reaktionsgase den statischen Mischer innerhalb einer Verweilzeit von ca. 0,1 s durchströmen. Die Reaktionstemperatur wird dabei auf 360°C eingestellt.

Selbstverständlich können die Phosgenierungen in der Gasphase auch nach einer anderen als der oben beschreibenen Methode durchgeführt werden.

Die bei der Phosgenierung von Monaminen in der Gasphase entstehenden Reaktionsgase werden in den genannten Lösungsmitteln absorbiert, ebenfalls nach bekannten Methoden des Standes der Technik. So können z.B. die nach oben strömenden Gase innerhalb eines Absorptionsgefäßes durch die oberhalb des Gaseintritts in das Gefäß eingegebenen und nach unten fließenden Lösungsmittel absorbiert werden. Dabei wird zweckmäßig als Absorptionsgefäß eine Kolonne verwendet, die vorteilhaft zusätzliche Produktkühler enthält, um die Reaktionsgase und die entstehenden Lösungen zu kühlen.

Bevorzugt wird die Absorption der Reaktionsgase in den Lösungsmitteln so durchgeführt, daß der bei der Gasphasenphosgenierung entstehende Chlorwasserstoff und das gegebenenfalls in den Reaktionsgasen enthaltene überschüssige Phosgen am oberen Ende des verwendeten Absorptionsgefäßes, beispielsweise am Kopf einer Absorptionskolonne, gasförmig entweichen.

Selbstverständlich kann die Absorption der genannten Reaktionsgase auch in einer anderen als der oben beschriebenen Weise durchgeführt werden.

Die bei der Absorption der Reaktionsgase in den genannten Lösungsmitteln entstehenden Lösungen können auch Phosgen enthalten. Die Konzentrationen an Phosgen in diesen Lösungen sind u.a. abhängig von dem in die Gasphasenphosgenierung eingesetzten Phosgenüberschuß, von den eingesetzten Lösungsmitteln und von der Methode, nach der die Reaktionsgase in den Lösungsmitteln absorbiert werden. Dementsprechend können die Konzentrationen an Phosgen in diesen Lösungen in einem weiten Bereich schwanken.

Um diese Lösungen gegebenenfalls von dem in ihnen enthaltenen Phosgen zu befreien, werden bekannte Methoden des Standes der Technik angewendet. So kann die genannte Abtrennung von Phosgen aus diesen Lösungen beispielsweise durch fraktionierte Destillation in einer trennwirksamen Kolonne durchgeführt werden.

Die erhaltenen von Phosgen befreiten flüssigen Gemische stellen im wesentlichen Lösungen der genannten Carbamidsäurechloride in den genannten Lösungsmitteln dar. Sie enthalten gegebenenfalls auch die genannten Monoisocyanate, deren Bildung verschiedene Ursachen haben kann. Beispielsweise enthalten die heißen Reaktionsgase bei der Gasphasenphosgenierung Isocyanate, die bei der nachfolgenden Absorption in einem Lösungsmittel evtl. nur unvollständig mit Chlorwasserstoff rekombinieren. Weiterhin können z.B. anteilig Isocyanate durch thermische Chlorwasserstoff-Abspaltung aus den entsprechenden Carbamidsäurechloriden gebildet werden, wenn die zugrunde liegenden Monoamine in einem Lösungsmittel am Rückfluß phosgeniert werden oder wenn Carbamidsäurechlorid enthaltende Lösungen zur befreiung von gelöstem Phosgen erhitzt werden. Dabei sind die Konzentrationen an Monoisocyanat in diesen Lösungen unter anderem von der Art des in die Phosgenierung eingesetzten Monoamins, der Art der verwendeten Lösungsmittel, der Verdünnung der Lösungen mit den genannten Lösungsmitteln, der verwendeten Methode zur Absorption der Reaktionsgase und der verwendeten Methode zur Abtrennung von gelöstem Phosgen abhängig.

Beim erfindungsgemäßen Verfahren können die gegebenenfalls bereits Monoisocyanat innerhalb der genannten Konzentrationen enthaltenden Lösungen ohne weitere Aufarbeitung eingesetzt werden. Es ist jedoch auch möglich, das in den Lösungen vorhandene Monoisocyanat in einem separaten Destillationsschritt zu entfernen, um beim erfindungsgemäßen Verfahren im wesentlichen

ausschließlich Carbamidsäurechlorid enthaltende Lösungen einzusetzen. Eine derartige Arbeitsweise wäre jedoch mit dem Problem einer selektiven destillativen Entfernung des Monoisocyanats belastet, so daß man im allgemeinen vor der Durchführung des erfindungsgemäßen Verfahrens auf eine derartige, im Prinzip überflüssige, Abtrennung des bereits vorhandenen Monoisocyanats verzichtet.

Die wesentliche, dem erfindungsgemäßen Verfahren zur Aufarbeitung der genannten technischen Lösungen zugrunde liegende Beobachtung bestand darin, daß aus einem, im wesentlichen aus Monoisocyanat, Carbamidsäurechlorid und Lösungsmittel bestehenden Gemisch durch thermische Spaltung des Carbamidsäurechlorids in Isocyanat und Chlorwasserstoff, sowie durch destillative Aufarbeitung dann das gewünschte Monoisocyanat in optimaler Ausbeute und Reinheit gewonnen werden kann, wenn man eine solche Mischung kontinieurlich, seitlich in eine Destillationskolonne einspeist, an deren Kopf das reine Monoisocyanat entnommen wird, gleichzeitig der Kolonne oberhalb der genannten Einspeisstelle einen im wesentlichen aus konzentrierter Carbamidsäurelösung bestehenden Seitenstrom entnimmt, diesen zumindest mit einem Teil des gegebenenfalls mit Carbamidsäurechlorid verunreinigten, am Sumpf der Kolonne anfallenden Lösungsmittel vereinigt und die vereinigten Ströme einem Spalter zuführt, wo sie in Chlorwasserstoff, Lösungsmittel und in die Kolonne zurückzuführende, Monoisocyanat, Carbamidsäurechlorid und Lösungsmittel aufweisende Lösung zerlegt werden.

Bei dem beschriebenen System handelt es sich somit um einen Kreislauf, welchem am Kopf der Kolonne reines Isocyanat und im Spalter Chlorwasserstoff sowie Lösungsmittel entzogen werden. Es genügt nun zur Durchführung des kontinuierlichen, erfindungsgemäßen Verfahrens, diesem Kreislauf an einer beliebigen Stelle eine solche Menge der aufzuarbeitenden technischen Lösung zuzuführen, die der Menge des anfallenden Monoisocyanats, Chlorwasserstoffs und Lösungsmittels entspricht.

Es gehört zwar zum bekannten Stand der Technik, Dreikomponentengemische durch fraktionierte Kolonnendestillation dergestelt aufzutrennen, daß man das niederigstsiedende Produkt am Kolonnenkopf, daß hochsiedende Produkt am Kolonnensumpf und das mittelsiedende Produkt aus einem Seitenstrom gewinnt; jedoch führt im allgemeinen eine derartige Destillation mit Seitenstromentnahme zu einer Reduzierung der Ausbeute an niedrigstsiedendem Kopfprodukt, da ein Teil des Kopfprodukts stets mit dem mittelsiedenden Produkt im Sietenstrom entnommen wird. Es muß daher als äußerst überraschend bezeichnet werden, daß bei der Durchführung des erfindungsgemäßen Verfahrens eine maximale Ausbeute an Kopfprodukt (Monoisocyanat) nur dann möglich wird, wenn, wie oben beschrieben, unter Entnahme eines Seitenstroms, Kombination des Seitenstroms mit dem Sumpf und weitere Behandlung der vereinigten Ströme verfahren wird.

Die Ausbeuten beim dergestalt arbeitenden erfindungsgemäßen Verfahren an einem Monoisocyanat sind wesentlich höher, als dies bei einer einfachen Destillation ohne Seitenstromentnahme möglich wäre.

Das erfindungsgemäße Verfahren sei im folgenden anhand der Zeichnungen näher erläutert. Dabei stellen die in den Figuren 1 bis 4 dargestellten Apparaturen lediglich bespielhaft zur Durchführung des erfindungsgemäßen Verfahrens geeignete Vorrichtungen dar. Das erfindungsgemäße Verfahren ist jedoch keineswegs auf die zwingende Verwendung der in den Figuren 1 bis 4 dargestellten Vorrichtungen beschränkt.

In Figur 1 bedeuten

(A) eine Destillationskolonne und

(B) einen Spalter, der im wesentlichen aus einem mittels Umlaufverdampfer (107) beheizbaren Reaktionsgefäß mit aufgesetztem Rückflußkühler (106) und unterhalb des Rückflußkühlers angeordnetem Entnahmeboden für das Kondensat (109) besteht.

Bei der Durchführung des erfindungsgemäßen Verfahrens gemäß einer ersten Ausführungsform in der in Fig. 1 dargestellten Apparatur wird die aufzuarbeitende, technische Lösung (101) in die Destillationskolonne A) eingespeist, an deren Kopf das reine Monoisocyanat (102) und an deren Sumpf das gegebenenfalls verunreinigte Lösungsmittel (103) anfallen. Oberhalb der Einspeisstelle für die Lösungen (101) wird ein Seitenstrom (104) entnommen, der mit dem aus dem Sumpf entnommenen Lösungsmittel (103) vereinigt wird. Die vereinigten Ströme (105) werden in das mit einem Schlangenkühler als Rückflußkühler (106) versehene Reaktionsgefäß (B) eingebracht und dort mit Hilfe des Umlaufverdampfers (107) aufgeheizt. Oberhalb des Rückflußkühlers (106) entweicht der entstehende Chlorwasserstoff (108), während am Rückflußkühler (106) sich bildendes Kondensat über einen Entnahmeboden 109 aus dem Reaktionsgefäß (B) entnommen und als Monoisocyanat, Carbamidsäurechlorid und Lösungsmittel enthaltende Lösung (110) in die Destillationskolonne (A) zurückgeführt werden. Gleichzeitig wird dem Reaktionsgefäß (B) gegebenenfalls verunreinigtes Lösungsmittel (111) als Flüssigkeit entnommen.

In Figur 2 bedeuten

(A) eine Destillationskolonne,

(A') eine mit der Destillationskolonne A) in Serie geschaltete weitere Destillationskolonne und

(B) einen mittels Durchlauferhitzer (209) beheizbaren Spalter, der mit einem Rückfluß-

kühler (210) verbunden ist.

Bei der Durchführung des erfindungsgemäßen Verfahrens gemäß einer denkbaren zweiten Ausführungsform in der in Fig. 2 dargestellten Apparatur wird das im, oberhalb des Spalters (B) angeordneten, Rückflußkühler (210) anfallende Monoisocyanat, Carbamidsäurechlorid und Lösungsmittel enthaltende Kondensat (201) in den oberan Teil der Destillationskolonne auf (A) eingespeist, die mit einer zweiten Kolonne (A') dergestalt verbunden ist, daß die Arbeitsweise der kombinierten Kolonnen (A) und (A') der Arbeitsweise einer einzigen Kolonne der in Fig. 1 dargestellten Art entspricht. Die am Kopf der Kolonne (A) anfallenden Dämpfe (202) werden demzufolge in den unteren Teil der Kolonne (A') eingegeben. Entsprechend wird die Sumpflösung (203) der Kolonne (A') in den Kopf der Kolonne (A) zurückgeführt. Während am Kopf der Kolonne (A') das reine Monoisocyanat anfällt, fallen am Sumpf der Kolonne (A) die gegebenenfalls verunreinigten Lösungsmittelströme (205) und (206) an. Aus dem Sumpf der Kolonne (A') wird ein Produktstrom (207) entnommen, welcher als Seitenstrom der aus den Kolonnen (A) und (A') bestehenden Destillation-seinheit betrachtet werden kann. Dieser "Seitenstrom" wird mit dem Sumpf-Teilstrom (206), der 20 bis 100% der Gesamtmenge des Sumpfprodukts der Kolonne (A) darstellen kann, vereinigt. Die vereinigten Ströme (208) werden mit der erfindungsgemäß aufzuarbeitenden technischen Lösung (213) in den Durchlauferhitzer (209) unter zumindest teilweisem Verdampfen erhitzt und in das mit einem Rohrbündelkühler als Rückflußkühler (210) versehene Reaktionsgefäß (B) eingebracht. Oberhalb des Rückflußkühlers (210) entweicht der entstehende Chlorwasserstoff (211) während das am Rückflußkühler anfallende Kondensat als Strom (201) in die Kolonne (A) zurückgeführt wird. Gleichzeitig wird dem Reaktionsgefäß (B) gegebenenfalls noch verunreinigtes Lösungsmittel (212) als Flüssigkeit entnommen.

In Figur 3 bedeuten

(A) eine Destillationskolonne und

(B) einen mittels einer Heizvorrichtung (307) beheizbaren, einen eingebauten Rückflußkühler (306), eine eingebaute Füllkörperpackung (308), einen eingebauten "Waschlösung-Entnahmeboden" (312) und einen Entnahme-boden (313) für das Kondensat des Rückflußkühlers aufweisenden Spalter.

Bei der Durchführung des erfindungsgemäßen Verfahrens gemäß einer denkbaren dritten Ausführungsform in der in Fig. 3 dargestellten Apparatur wird das am Rückflußkühler (306) anfallende, im Entnahmeboden (313) gesammelte, Monoisocyanat, Carbamidsäurechlorid und Lösungsmittel enthaltende Kondensat (301) in die Destillationskolonne (A) eingespeist, an deren Kopf das reine Monoisocyanat (302) und an deren Sumpf das gegebenenfalls noch verunreinigte Lösungsmittel (303) anfallen. Oberhalb der Einspeisstelle für die Lösung (301) wird en Seitenstrom (304) entnommen. Dieser Seitenstrom (304), sowie ein Teil (305) des aus Sumpf der Kolonne (A) entnommen Lösungsmittels (303) und die aufzaurbeitende technische Lösung (315) werden in den beheizbaren Spalter (B) eingebracht, dort vereinigt und mittels der Heizung (307) unter teilweiser Verdampfung und teilweiser Spaltung des Carbamidsäurechlorids erhitzt. Der oberhalb des Rückflußkühlers (306) entweichende Chlorwasserstoff wird in der Füllkörperpackung (308) mit einem weiteren Teil (309) des aus dem Sumpf der Kolonne (A) entnommenen Lösungsmittels (303) gewaschen und entweicht dem Spalter (B) in praktisch reiner Form (310). Die entstehende Waschlösung (311) wird über einen Entnahmeboden (312) in den unteren Teil des Reaktionsgefäßes geleitet. Das sich am Rückflußkühler (306) bildende Kondensat wird über einen Entnahmeboden (313) dem Reaktionsgefäß entnommen und in die Destillationskolonne (A) zurückgeführt. Gleichzeitig wird dem Reaktionsgefäß, d.h. dem Spalter (B) gegebenenfalls verunreinigtes Lösungsmittel (314) als Flüssigkeit entzogen.

In Figur 4 bedeuten

(A) eine Destillationskolonne und

(B) einen mittels eines Umlaufverdampfers (409) beheizbaren, eine Füllkörperpackung (408) aufweisenden, mit einem Rückflußkühler (407) verbundenen Spalter.

Bei der Durchführung des erfindungsgemäßen Verfahren gemäß einer möglichen vierten Ausführungsform in der in Fig. 4 dargestellten Apparatur wird die aufzaurbeitende technische Lösung (401) wie bei der ersten Ausführungsform in die Destillationskolonne (A) eingespeist, an deren Kopf das reine Monoisocyanat (402) und an deren Sumpf das gegebenenfalls verunreinigte Lösungsmittel (403) anfallen. Oberhalb der Einspeisstelle für die Lösung (401) wird ein Seitenstrom (404) entnommen und mit einem Teil (405) des auf dem Sumpf der Kolonne (A) entnommenen Lösungsmittels (403) vereinigt. Die vereinigten Ströme (406) werden in das mit einem Schlangenkühler als Rückflußkühler (407) versehene Reaktionsgefäß (B) eingebracht, das unterhalb der Einspeisstelle für diese Ströme eine trennwirksame Füllkörperpackung (408) enthält. Die Sumpfflüssigkeit des Reaktionsgefäßes (B) wird mit Hilfe eine Umlaufverdampfers (409) aufgeheizt. Oberhalb des Rückflußkühlers (407) entweicht der entstehende Chlorwasserstoff (410). Das sich am Rückflußkühler (407) bildende Monoisocyanat, Carbamidsäurechlorid un Lösungsmittel enthaltende Kondensat wird in die Kolonne (A) zurückgegeben (411). Ein weiterer Teil (412) des aus dem Sumpf der Kolonne (A) entnommen Lösungsmittels (403) wird in den Sumpf des Reaktionsgefäßes (B) eingebracht. Gleichzeitig

wird dem Reaktionsgefäß (B) gegebenenfalls verunreinigtes Lösungsmittel (413) als Flüssigkeit entnommen.

Allen Ausführungsformen des erfindungsgemäßen Verfahrens gemeinsam, da erfindungswesentlich, ist die Entnahme eines Seitenstroms oberhalb der Einspeisstelle der Destillationskolonne, die Vereinigung dieses Seitenstroms mit zumindest einem Teil des im Sumpf der Destillationskolonne anfallenden Lösungsmittels, die zumindest teilweise Zerlegung der vereinigten Ströme in Lösungsmittel, Chlorwasserstoff und in die Kolonne rückzuführendes Kondensat, sowie die Einspeisung der aufzaarbeitenden technischen Lösung an einer beliebigen Stelle dieses Kreislaufs. Für die Durchführung des erfindungsgemäßen Verfahrens ist es nicht wesentlich, ob die aufzuarbeitenden, bei der Phosgenierung der genannten Monoamine anfallenden, technischen Lösungen in die Destillationskolonne oder in das Reaktionsgefäß eingespeist werden. Zweckmäßig richtet sich jedoch die Einspeisung nach der Zusammensetzung der Ausgangslösungen, die in einem weiten Bereich schwanken kann. So ist es vorteilhaft, Lösungen mit einem hohen Gehalt an Monoisocyanaten in die Destillationskolonne, dagegen Lösungen mit einem niedrigen Gehalt an Monoisocyanat in das Reaktionsgefäß einzuspeisen.

Das Gewichtsverhältnis der in den Kreislauf eingespeisten, erfindungsgemäß aufzuarbeitenden technischen Lösung zu der im Kreislauf befindlichen Produktmenge liegt bei allen Ausführungsformen des erfindungsgemäßen Verfahrens zwischen 1:0,1 und 1:20, vorzugsweise 1:0,2 und 1:10.

Der vom Rückflußkühler zur Kolonne zurückgeführte Strom enthält im allgemeinen 1 bis 30, vorzugsweise 3 bis 20 Gew.-% Monoisocyanat und 0,5 bis 30, vorzugsweise 2 bis 25 Gew.-% Carbamidsäurechlorid.

Der der Kolonne entnommene Seitenstrom enthält im allgemeinen 0,1 bis 20, vorzugsweise 0,2 bis 10 Gew.-% Monoisocyanat und 20 bis 80 vorzugsweise 30 bis 65 Gew.-% Carbamidsäurechlorid.

Das im Sumpf der Destillationskolonne anfallende Lösungsmittel enthält 0 bis 3 vorzugsweise 0 bis 1 Gew.-% Carbamidsäurechlorid und 0 bis 3 vorzugsweise 0 bis 1 Gew.-% Monoisocyanat.

Wie bereits bei der Schilderung der zweiten, dritten und vierten Ausführungsform des erfindungsgemäßen Verfahren erläutert, ist es nicht erforderlich, die Gesamtmenge des Kolonnensumpfes mit dem Seitenstrom zu vermischen.

Es ist im allgemeinen ausreichend, nur einen solchen Teil des Sumpfprodukts zur Rückvermischung mit dem Seitenstrom zu verwenden, der zur Einstellung eines Gehalts der vereinigten Lösung an Carbamidsäurechlorid von 1 bis 30 vorzugsweise 3 bis 25 Gew.-% erforderlich ist.

Die dem Reaktionsgefäß bzw. Spalter in flüssiger Form entnommenen Lösungsmittel enthalten im allgemeinen 0 bis 3 vorzugsweise 0 bis 1 Gew.-% Carbamidsäurechlorid und 0 bis 3 vorzugsweise 0 bis 1 Gew.-% Monoisocyanat.

Der Anteil des als Sumpfprodukt der Destillationskolonne anfallenden Lösungsmittels, welcher nicht zur Rückvermischung mit dem Seitenstrom benötigt wird, kann entweder wie bei der Schilderung der dritten Ausführungsform erläutert als Waschflüssigkeit für den den Rückflußkühler verlassenden Chlorwasserstoff dienen oder in den Sumpf des Spalters (4. Ausführungsform) geleitet werden. Es ist auch möglich, dem System einen Teil des anfallenden Lösungsmittels aus dem Sumpf der Kolonne zu entnehmen, wie das andeutungsweise bei der Schilderung der 2. Ausführungsform erläutert wird.

Die bei der Durchführung des erfindungsgemäßen Verfahrens gegenbenenfalls noch mit Monoisocyanat und/oder Carbamidsäurechlorid verunreinigten Lösungsmittel können verteilhaft destillativ beispielsweise in einer Abstreifkolonne gereinigt werden, wobei einerseits die gereinigten Lösungsmittel und andererseits Monoisocyanate und/oder Carbamidsäurechloride gegebenenfalls im Gemisch mit Lösungsmittel gewonnen werden. Die letztgenannten Destillationsprodukte werden zweckmäßig in das Reaktionsgefäß oder in die Destillationskolonne mit eingespeist. Die beim erfindungsgemäßen Verfahren anfallenden Lösungsmittel können nach Reinigung oder auch ohne vorhergehende Reinigung auch als Ausgangslösungmittel zur Herstellung der erfindungsgemäß aufzuarbeitenden technischen Lösungen verwendet werden.

Das erfindungsgemäße Verfahren ist nicht auf die beispielhaft erläuterten Ausführungsformen beschränkt. So können zur destillativen Aufarbeitung, wie dies bei der Schilderung der 2. Ausführungsform bereits angedeutet worden ist, auch mehrere Kolonnen verwendet werden, wenn sie so hintereinandergeschaltet sind, daß ihre technische Funktion der Funktion einer einzigen Kolonne entspricht.

Die Entnahme des Seitenstroms aus der Destillationskolonne erfolgt "oberhalb" der Einspeisstelle für die Ausgangslösungen, was im Falle der Verwendung mehrerer, im Verbund befindlicher Kolonnen bedeutet, daß der genannte Seitenstrom im Sinne der Trennwirkung zwischen der Einspeisstelle für die vom Spalter zurückgerführte Lösung und gegebenenfalls für die Ausgangslösung und der Entnahmestelle für das reine Monoisocyanat entnommen wird.

Das erfindungsgemäße Verfahren wird unter Verwendung der in der chemischen Technologie an sich bekannten Apparaturen durchgeführt.

Als Rückflußkühler können beispielsweise an sich bekannte Schlangenkühler oder Rohrbündelkühler Verwendung finden.

Zum Aufheizen der in das Reaktionsgefäß

(Spalter) geführten Produktströme können an sich bekannte Durchlauferhitzer verwendet werden. Die Flüssigkeiten können auch beispielsweise innerhalb des Reaktionsgefäßes mit Hilfe von im Bodenteil befindlichen Mantelheizungen, Einsteckverdampfern oder Umlaufverdampfern aufgeheizt werden.

Als Reaktionsgefäß können in der chemischen Technologie gebräuchliche Behälter verwendet werden. Es ist für die Durchführung des erfindungsgemäßen Verfahrens nicht wesentlich, den Rückflußkühler innerhalb des Reaktionsgefäßes anzuorden. Der Rückflußkühler kann veilmehr auch über entsprechende Leitungen mit dem Reaktionsgefäß verbunden sein. Besonders vorteilhaft kann es sein, als Reaktionsgefäß trennwirksame Kolonnen zu benutzen. Hierbei wird eine gleichzeitige Chlorwasserstoffabspaltung und -abtrennung und Reinigung des verwendeten Lösungsmittels durch fraktionierte Destillation in einem Apparat ermöglicht (vgl. 4. Ausführungsform).

Die Entnahme zumindest eines Teils des sich am Rückflußkühler bildenden Kondensats erfolgt nach bekannten Methoden des Standes der Technik beispielsweise mittels eines unterhalb des Rückflußkühlers befindlichen Entnahmebodens. Bei Verwendung eines außserhalb des Reaktionsgefäßes angeordneten Rückflußkühlers kann das Kondensat auch beispielsweise am Boden dieses Kühlerapparates aufgefangen und von dort entnommen werden.

Als Destillationskolonne werden an sich bekannte Apparate des Standes der Technik mit einem Entnahmeboden für den Seitenstrom verwendet.

Bei der Durchführung des erfindungsgemäßen Verfahrens kann in allen Apparaturen sowohl unter Unterdruck, als auch unter Normaldruck, als auch unter Überdruck gearbeitet werden. Im allgemeinen herrscht in den Apparaturen ein Druck von 0,01 bis 10 bar. Die Druckbedingungen hängen selbstverständlich von der Temperatur und der Flüchtigkeit der einzelnen Komponenten ab.

Die Temperatur im Spalter liegt im allgemeinen zwischen 30 und 250 vorzugsweise 80 und 160°C. Im allgemeinen wird die Temperatur so bemessen, daß 5 bis 95% vorzugsweise 10 bis 65% der eingetragenen Flüssigkeit unter zumindest teilweiser Spaltung des Carbamidsäurechlorids verdampfen.

Bei der Durchführung des erfindungsgemäßen Verfahrens wird die Hauptdestillationskolonne im allgemeinen unter einem Rücklaufverhältnis von 1:1 bis 1:30 betrieben.

Das erfindungsgemäße Verfahren weist gegenüber den bekannten Verfahren des Standes der Technik insbesondere folgende Vorteile auf:

1. Zur Durchführung des Verfahrens werden keine zusätzlichen Stoffe, wie Chlorwasserstoffakzeptoren oder Hydroxyl-Verbindungen, benötigt. Dementsprechend fallen bei der Durchführung des Verfahrens weder gesonderte Reaktionsschritte noch speziell aufzuarbeitende Nebenoder Folgeprodukte an.
2. Bei der Durchführung des Verfahrens werden nur solche Apparate verwendet, die in der Technik bekannt und gebräuchlich sind, z.B. Kolonnen und Wärmetauscher. Komplizierte und schwer herzustellende Apparate werden dazu nicht benötigt. Insbesondere die erfindungswesentliche Entnahme eines Seitenstroms aus der Destillationskolonne kann mit Hilfe der gebräuchlichen Methoden des Standes der Technik bewerkstelligt werden.
3. Das technisch einfach durchzuführende erfindungsgemäße Prinzip der Seitenstromentnahme mit der Rückvereinigung der Ströme bedingt gleichermaßen eine wesentlich effektivere Gewinnung der Monoisocyanate und eine wesentlich effektivere Chlorwasserstoff-Abspaltung aus den Carbamidsäurechloriden, so daß zur Gewinnung der Monoisocyanate weit weniger Produktkreisläufe erforderlich sind. Das hat zur Folge, daß der spezifische Energieverbrauch beteutend niedriger ist und die Raum-Zeit-Ausbeute von Produktionsanlagen erheblich ansteigt.
4. Aufgrund der niedrigen Rücklaufverhältnisse in der Destillationskolonne und der geringen Anzahl von Produktkreis-läufen ergibt sich bei dem erfindungsgemäßen Verfahren eine wesentlich geringere thermische Belastung der wärmeempfindlichen Monoisocyanate und Carbamidsäurechloride. Dadurch werden nur sehr kleine Anteile dieser Substanzen zu Nebenprodukten umgesetzt, so daß die Monoisocyanate in praktisch quantitativer Ausbeute gewonnen werden können.
5. Das erfindungsgemäße Verfahren gestattet es, gewünschtenfalls gleichzeitig in einem Apparat die Chlorwasserstoff-Abspaltung und die Lösungsmittel-Reinigung durchzuführen. Dabei werden sowohl Investitionskosten als auch Energiekosten eingespart (vgl. 4 Ausführungsform).

Die nach dem erfindungsgemäßen Verfahren herstellbaren Alkylmonoisocyanate sind wertvolle Ausgangsverbindungen für Pflanzenschutzmittel und Pharmazeutika.

In den nachfolgenden Beispielen wurde jeweils eine 6 m hohe, mittels eines Umlaufverdampfers beheizbare Füllkörperkolonne (Nennweite der unteren 2 m:100 mm, Nennweite der oberen 4 m:80 mm) verwendet. Die Kolonne wurde jeweils unter einem Rücklaufverhältnis von 1:8 betrieben. Die Einspeisstellen befanden sich jeweils in einer Höhe von 2 m, während die Seitenstromentnahme in einer Höhe von 3 m erfolgte.

Alle Prozentangaben beziehen sich auf Gewichtsprozente.

Beispiel 1 (Figur 1)

Es wird eine technische Lösung von 2,01% Methylisocyanat und 6,33% Methylcarbamidsäurechlorid in Chlorbenzol kontinuierlich aufgearbeitet. 48,96 kg/h dieser Lösung werden in die Destillationskolonne (A) (Sumpftemperatur 136°/1,2 bar) eingespeist (101), an deren Kopf 2,74 kg/h reines Methylisocyanat (102) und an deren Sumpf (103) 98,35 kg/h Produkt (0% Methylisocyanat; 0,39% Methylcarbamidsäurechlorid) entnemmen werden. Als Seitenstrom (104) werden 18,43 kg/h Lösung (6,09% Methylisocyanat; 51,73% Methylcarbamidsäurechlorid) entnommen und mit dem Sumpfprodukt (103) vereinigt. Die vereinigten Ströme (116,8 kg/h; 0,96% Methylisocyanat; 8,50% Methylcarbamidsäurechlorid) werden in das Reaktionsgefäß (B) eingebracht (105) und dort durch Erhitzen auf 128°C/1,0 bar teilweise verdampft. Oberhalb des Rückflußkühlers (106) entweichen 1,17 kg/h Chlorwasserstoff (108). Über den Entnahmeboden (109) werden 70,56 kg/h Kondensat (4,07% Methylisocyanat; 9,67% Methylcarbamidsäurechlorid) entnommen und in die Destillationskolonne (A) eingespeist (110). Aus dem Sumpf des Reaktionsgefäßes (B) werden 45,05 kg/h Flüssigkeit (0,18% Methylisocyanat; 0,20% Methylcarbamidsäurechlorid) entnommen (111).

Beispiel 2 (Figur 3)

Es wird eine technische Lösung von 0,41% Methylisocyanat und 7,32% Methylcarbamidsäurechlorid in Chlorbenzol kontinuierlich aufgearbeitet. 67,70 kg/h dieser Lösung werden in das Reaktionsgefäß (B) eingebracht und teilweise verdampft. Über den Entnahmeboden (313) werden 88,30 kg/h Kondensat (4,07% Methylisocyanat; 9,57% Methylcarbamidsäurechlorid) entnommen und in die Destillationskolonne (A) (Sumpftemperatur: 135°C/1,2 bar) eingespeist (301). Am Kopf der Kolonne werden 2,79 kg/h reines Methylisocyanat (302), am Sumpf (303) der Kolonne werden 70,55 kg/h Flüssigkeit (0% Methylisocyanat; 0,99% Methylcarbamidsäurechlorid), und über den Seitenstrom (304) werden 14,96 kg/h Lösung (5,35% Methylisocyanat; 51,82% Methylcarbamidsäurechlorid) gewonnen. Der Seitenstrom (304) und 56,69 kg/h von dem entnommenen Sumpfprodukt (303) der Kolonne (305) werden in den unteren Teil des Reaktionsgefäßes (B) eingebracht und dort bei 130°C 1,1 bar teilweise verdampft. Der oberhalb des Rückflußkühlers (306) entweichende Gasstrom wird in der Füllkörperpackung (308) mit 13,86 kg/h flüssigem Sumpfprodukt der Kolonne (A) gewaschen, die oberhalb der Packung (308) in das Reaktionsgefäß eingegeben werden (309). Die ablaufende Waschlösung (14,43 kg/h; 0% Methylisocyanat; 4,88% Methylcarbamidsäurechlorid) wird über den Entnahmeboden (312) in den unteren Teil des Reaktionsgefäßes (B) eingegeben (311). Am Kopf des Reaktionsgefäßes entweichen 1,78 kg/h Chlorwasserstoff-Gas (310). Am Boden des Reaktionsgefäßes (314) werden 63,12 kg/h Flüssigkeit (0,44% Methylisocyanat; 0,60% Methylcarbamidsäurechlorid) entnommen.

Beispiel 3 (Figur 4)

Es wird eine technische Lösung von 2,70% Methylisocyanat und 8,72% Methylcarbamidsäurechlorid in Chlorbenzol kontinuierlich aufgearbeitet. 36,64 kg/h dieser Lösung werden in die Destillationskolonne (A) (Sumpftemperatur: 136°C/1,2 bar) eingespeist (401), an deren Kopf 2,91 kg/h reines Methylisocyanat (402) und an deren Sumpf (403) 55,81 kg/h Produkt (0% Methylisocyanat; 0,66% Methylcarbamidsäurechlorid) gewonnen werden. Als Seitenstrom (404) werden 18,12 kg/h Lösung (5,62% Methylisocyanat; 50,90% Methylcarbamidsäurechlorid) aus der Kolonne entnommen und mit 27,91 kg/h des Sumpfproduktes der Kolonne (405) vereinigt. Die vereinigten Flüssigkeiten (46,03 kg/h; 2,21% Methylisocyanat; 20,43% Methylcarbamidsäurechlorid) werden oberhalb der Packung (408) in das Reaktionsgefäß (B) eingebracht (406) und dort teilweise verdampft. Die Beheizung erfolgt durch die durch die Packung (408) aufsteigenden Lösungsmitteldämpfe, die ihrerseits durch Erhitzen des Sumpfes des Reaktionsgefäßes bei 135°C/1,1 bar mittels des Umlaufverdampfers (409) erhalten werden. Oberhalb des Rückflußkühlers (407) entweichen 1,23 kg/h Chlorwasserstoff-Gas (410). Das vom Rückflußkühler (407) ablaufende Kondensat (40,19 kg/h; 7,31% Methylisocyanat; 15,90% Methylcarbamidsäurechlorid) wird in die Destillationskolonne (A) zurückgegeben (411). 27,90 kg/h des aus der Destillationskolonne (A) entnommenen Sumpfproduktes (403) werden in den Sumpfteil des Reaktionsgefäßes (B) eingebracht (412), aus dem 32,51 kg/h Flüssigkeit (0% Methylisocyanat; 0,15% Methylcarbamidsäurechlorid) entnommen werden (413).

Beispiel 4 (Figur 4)

Es wird eine technische Lösung von 6,07% Ethylisocyanat und 4,03% Ethylcarbamidsäurechlorid in Chlorobenzol kontinuierlich aufgearbeitet. 33,62 kg/h dieser Lösung werden in die Destillationskolonne (A) (Sumpftemperatur: 136°C/1,2 bar) eingespeist (401), an deren Kopf 2,91 kg/h reines Ethylisocyanat (402) und an deren Sumpf (403) 42,83 kg/h Produkt (0% Ethylisocyanat; 0,17% Ethylcarbamidsäurechlorid) gewonnen werden. Als Seitenstrom (404) werden 7,68 kg/h Lösung (18,21% Ethylisocyanat; 31,58% Ethylcarbamidsäurechlorid) aus der Kolonne entnommen und mit 10,71 kg/h des Sumpfproduktes der Kolonne (405) vereinigt. Die vereinigten Flüssigkeiten (18,39 kg/h; 7,61% Ethylisocyanat; 13,30% Ethylcarbamidsäurechlorid werden oberhalb der Packung (408) in das Reaktionsgefäß (Sumpftemperatur: 136°C/1,1 bar) (B) eingebracht (406) und dort teilweise verdampft. Oberhalb des Rückflußkühlers (407) entweichen 0,45 kg/h Chlorwasserstoff-Gas (410). Das vom Rückflußkühler (407) ablaufende Kondensat (19,80

kg/h; 11,48% Ethylisocyanat; 5,79% Ethylcarbamidsäurechlorid) wird in die Destillationskolonnee (A) zurückgegeben (411). 32,12 kg/h des aus der Destillationskolonne (A) entnommenen Sumpfproduktes (403) werden in den Sumpfteil des Reaktionsgefäßes (B) eingebracht (412), aus dem 30,26 kg/h Flüssigkeit (0% Ethylisocyanat; 0,09% Ethylcarbamidsäurechlorid) entnommen werden (413).

**Patentansprüche**

1. Verfahren zur kontinuierlichen Aufarbeitung von technischen Lösungen, wie sie bei der Phosgenierung von Monoaminen der Formel

$$R—NH_2$$

in welcher
R für einen gegebenenfalls ungesättigten Alkylrest mit 1 bis 3 Kohlenstoffatomen steht, anfallen, und welche im wesentlichen aus Carbamidsäurechloriden der Formel

$$R—NH—CO—Cl$$

gegebenenfalls Monoisocyanaten der Formel

$$R—NCO$$

wobei
R die obengenannte Bedeutung hat,
und inerten, oberhalb 80°C und mindestens 10°C über dem Siedepunkt des Isocyanats R—NCO siedenden Lösungsmitteln bestehen, unter Gewinnung des reinen Monoisocyanats R—NCO durch thermische Spaltung des entsprechenden Carbamidsäurechlorids und Destillation des dadurch gewonnenen und des gegebenenfalls bereits vorliegenden Isocyanats, dadurch gekennzeichnet, daß man die aufzuarbeitende Lösung an einer beliebigen Stelle in einen Produktkreislauf einspeist, der dadurch hergestellt worden ist, daß

a) man eine Lösung, welche im wesentlichen aus dem zu gewinnenen Monoisocyanat, dem zu spaltenden Carbamidsäurechlorid und dem genannten Lösungsmittel besteht, oberhalb des Kolonnensumpfes in eine aus einer oder mehreren Kolonnen bestehenden Destillationseinheit einspeist, an deren Kopf das reine Monoisocyanat und an deren Sumpf das gegebenenfalls noch verunreinigte Lösungsmittel anfallen, wobei im Falle der Verwendung einer aus mehreren Kolonnen bestehenden Destillationseinheit den Kolonnen so hintereinander geschaltet sind, daß ihre technische Funktion der Funktion einer einzigen Kolonne entspricht,
b) man der Destillationseinheit oberhalb der in a) genannten Einspeisstelle einen flüssigen Produktstrom entnimmt, der im wesentlichen aus einer konzentrierten, gegebenenfalls Monoisocyanat enthaltenden Carbamidsäurechlorid-Lösung in den genannter Lösungsmitteln besteht, und diesen Produktstrom mit zumindest einem Teil des gemäß a) erhaltenen Sumpfprodukts vereinigt,
c) man die gemäß b) erhaltenen, vereinigten Ströme in ein ein mit einem Rückflußkühler versehenes Reaktionsgefäß einbringt, oder die in b) genannte Vereinigung innerhalb des genannten Reaktionsgefäßes durchführt, wobei man durch Erhitzen der Ströme vor und/oder nach ihrer Vereinigung, bzw. vor und/oder nach dem Einbringen in das Reaktionsgefäß für ein zumindest teilweises Verdampfen der flüssigen Phase unter zumindest teilweiser Spaltung des Carbamidsäurechlorids in Monoisocyanat und Chlorwasserstoff Sorge trägt,
d) man den dabei entstehenden Chlorwasserstoff oberhalb des Rückflußkühlers gasförmig entweichen läßt und gleichzeitig zumindest einen Teil des sich am Rückflußkühler bildenden Kondensats als gemäß a) in die Destillationseinheit einzuspeisende Lösung an den Kreislaufanfang gemäß a) zurückführt, wobei man schließlich dem in c) genannten Reaktionsgefäß gleichzeitig kontinuierlich gegebenenfalls verunreinigtes Lösungsmittel als Flüssigkeit entnimmt.

2. Verfahren gemäß Anspruch 1, dadurch gekennzeichnet, daß man die kontinuierlich aufzuarbeitende Lösung an der gleichen oder einer verschiedenen Stelle wie die gemäß a) in die Destillationseinheit einzuspeisende Lösung in die gleiche Destillationseinheit oberhalb deren Sumpf und unterhalb den Entnahmestelle des Seitenstroms gemäß b) einspeist.

3. Verfahren gemäß Anspruch 1, dadurch gekennzeichnet, daß man die aufzaurbeitende technische Lösung in das Reaktionsgefäß gegebenenfalls nach vorhergehendem Durchmischen mit einem in das Reaktionsgefäß einzubringenden Strom, einspeist.

4. Verfahren gemäß Anspruch 1 bis 3, dadurch gekennzeichnet, daß man als aufzuarbeitende technische Lösungen solche verwendet, wie sie bei der großtechnischen Phosgenierung von Methylamin anfallen.

**Claims**

1. A process for continuously working up technical solutions of the type which accumulate during the phosgenation of monoamines of the formula

$$R—NH_2$$

wherein
R represents an optionally unsaturated alkyl radical containing from 1 to 3 carbon atoms; and which consist essentially of carbamic acid chlorides of the formula

$$R—NH—CO—Cl$$

optionally monoisocyanates of the formula

R—NCO

wherein
R is defined as above;
and inert solvents boiling above 80°C and at least 10°C above the boiling point of the isocyanate R—NCO, with recovery of the pure monoisocyanate R—NCO by thermally decomposing the corresponding carbamic acid chloride and distilling the isocyanate obtained and the isocyanate already present, if any, characterised in that the solution to be worked up is introduced at any point into a product circuit which has been established

a) by introducing a solution consisting essentially of the monoisocyanate to be recovered, the carbamic acid chloride to be decomposed and the above-mentioned solvent into a distillation unit consisting of one or more columns, at the head of which the pure monoisocyanate and at the sump of which the optionally still contaminated solvent accumulate, at a point situated above the column sump; if a distillation unit is used consisting of several columns the columns are arranged one behind the other in such a way that their technical function corresponds to the function of a single column,
b) by removing from the distillation unit, at a point situated above the inlet mentioned in a), a liquid product stream consisting essentially of a concentrated carbamic acid chloride solution, optionally containing monoisocyanate, in the above-mentioned solvents, and combining this product stream with at least part of the sump product obtained in accordance with a);
c) by introducing the combined streams obtained in accordance with b) into a reaction vessel provided with a reflux condenser, or by effecting the combination mentioned in b) within the above-mentioned reaction vessel, provision being made by heating the streams before and/or after their combination or before and/or after their introduction into the reaction vessel, to ensure that the liquid phase is at least partly evaporated with at least a partial decomposition of the carbamic acid chloride into monoisocyanate and hydrogen chloride; and
d) by allowing the hydrogen chloride formed to escape as a gas above the reflux condenser and, at the same time, returning at least part of the condensate forming in the reflux condenser to the beginning of the circuit according to a) as the solution to be introduced into the distillation unit in accordance with a), optionally contaminated solvent finally being continuously removed at the same time as a liquid from the reaction vessel mentioned in c).

2. A process as claimed in Claim 1 characterised in that the solution to be continuously worked up is introduced at the same point as, or at a different point from; the solution to be introduced into the distillation unit in accordance with a) into the same distillation unit above its sump and below the point at which the side stream is removed in accordance with b).

3. A process as claimed in Claim 1, characterised in that the technical solution to be worked up is introduced into the reaction vessel optionally after admixture with a stream to be introduced into the reaction vessel.

4. A process as claimed in Claims 1 to 3, characterised in that solutions of the type accumulating during the phosgenation of methylamine on an industrial scale are used as the technical solutions to be worked up.

**Revendications**

1. Procédé de traitement en continu de solutions techniques telles que celles formées lors de la phosgénation de monoamines de formule:

$$R—NH_2$$

dans laquelle
R représente un groupe alkyle éventuellement insaturé contenant 1 à 3 atomes de carbone, et qui sont constituées essentiellement de chlorures d'acide carbamique de formule:

R—NH—CO—Cl

éventuellement de monoisocyanates de formule:

R—NCO

où
R a la signification indiquée ci-dessus,
ainsi que de solvants inertes d'un point d'ébullition supérieur de 80°C et d'au moins 10°C au point d'ébullition de l'isocyanate R—NCO, en obtenant le monoisocyanate pur R—NCO par dissociation thermique du chlorure d'acide carbamique correspondant et distillation de l'isocyanate ainsi obtenu et de l'isocyanate éventuellement déjà présent, caractérisé en ce qu'on charge la solution à traiter à n'importe quel endroit d'un circuit de produit que l'on établit comme suit:

a) on introduit une solution constituée essentiellement du monoisocyanate à obtenir, du chlorure d'acide carbamique à dissocier et du solvant mentionné au-dessus du fond de la ou des colonnes d'une unité de distillation constituée d'une ou de plusieurs colonnes et au sommet de laquelle on obtient le monoisocyanate pur et au fond de laquelle on obtient le solvant qui est encore éventuellement contaminé et, lorsqu'on utilise une unité de distillation constituée de plusieurs colonnes, celles-ci se succèdent de telle sorte

que leur fonction technique corresponde à la fonction d'une seule colonne,

b) de l'unité de distillation et audesses du point d'introduction mentionné sub (a), on retire un courant de produit liquide qui est essentiellement constitué d'une solution concentrée de chlorure d'acide carbamique contenant éventuellement le monoisocyanate dans les solvants mentionnés et l'on réunit ce courant de produit avec au moins une partie du produit de fond obtenu suivant (a),

c) on introduit les courants réunis obtenus suivant (b) dans un récipient réactionnel équipé d'un réfrigérant à reflux, ou on effectue la réunion mentionnée sub (b) à l'intérieur du récipient réactionnel mentionné en veillant à assurer, par chauffage des courants avant et/ou après leur réunion ou avant et/ou après leur introduction dans le récipient réactionnel, une évaporation au moins partielle de la phase liquide avec dissociation au moins partielle du chlorure d'acide carbamique en monoisocyanate et en chlorure d'hydrogène,

d) on laisse s'échapper, à l'état gazeux et audessus du réfrigérant à reflux, le chlorure d'hydrogène ainsi formé et, en même temps, on recycle, au début du circuit suivant (a), au moins une partie du condensat se formant dans le réfrigérant à reflux comme solution devant être introduite suivant (a) dans l'unité de distillation et enfin, du récipient réactionnel mentionné sub (c), on retire en même temps continuellement, comme liquide, le solvant éventuellement contaminé.

2. Procédé suivant la revendication 1, caractérisé en ce qu'on introduit la solution devant être soumise à un traitement continu au même endroit ou à un endroit différent de celui où la solution doit être introduite dans l'unité de distillation suivant (a), dans la même unité de distillation, au-dessus du fond de celle-ci et en dessous du point de prélèvement du courant latéral suivant (b).

3. Procédé suivant la revendication 1, caractérisé en ce qu'on introduit la solution technique à traiter dans le récipient réactionnel éventuelement après mélange préalable avec un courant devant être chargé dans le récipient réactionnel.

4. Procédé suivant l'une quelconque des revendications 1 à 3, caractérisé en ce que, comme solutions techniques à traiter, on utilise celles obtenues lors de la phosgénation de la méthylamine à l'échelle industrielle.

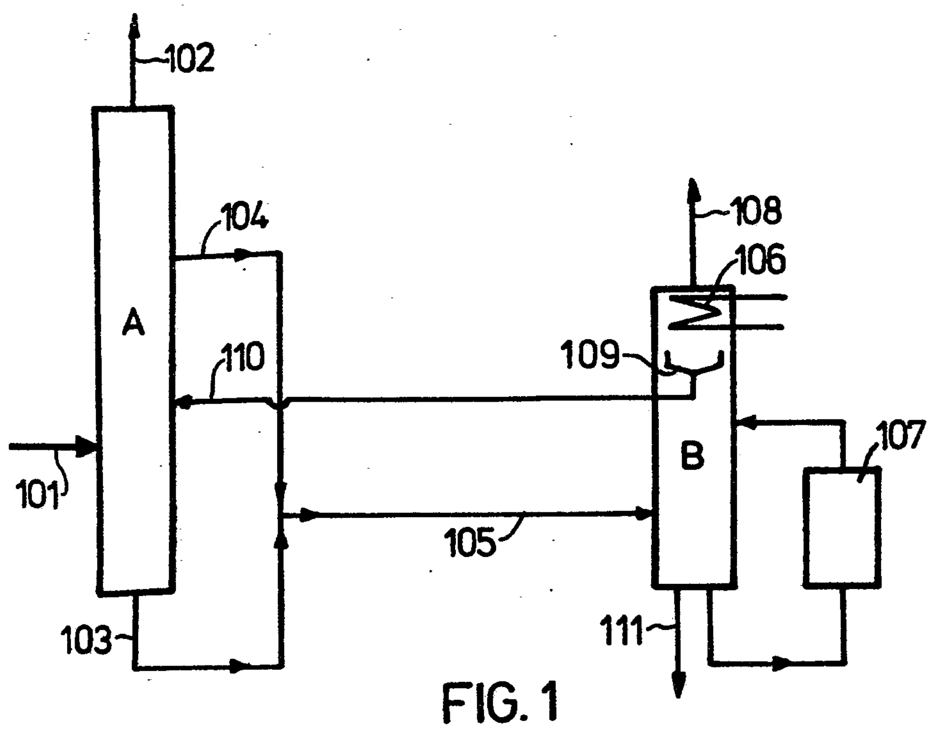
102
104
108
106
A
110
109
B
107
101
105
103
111

FIG. 1

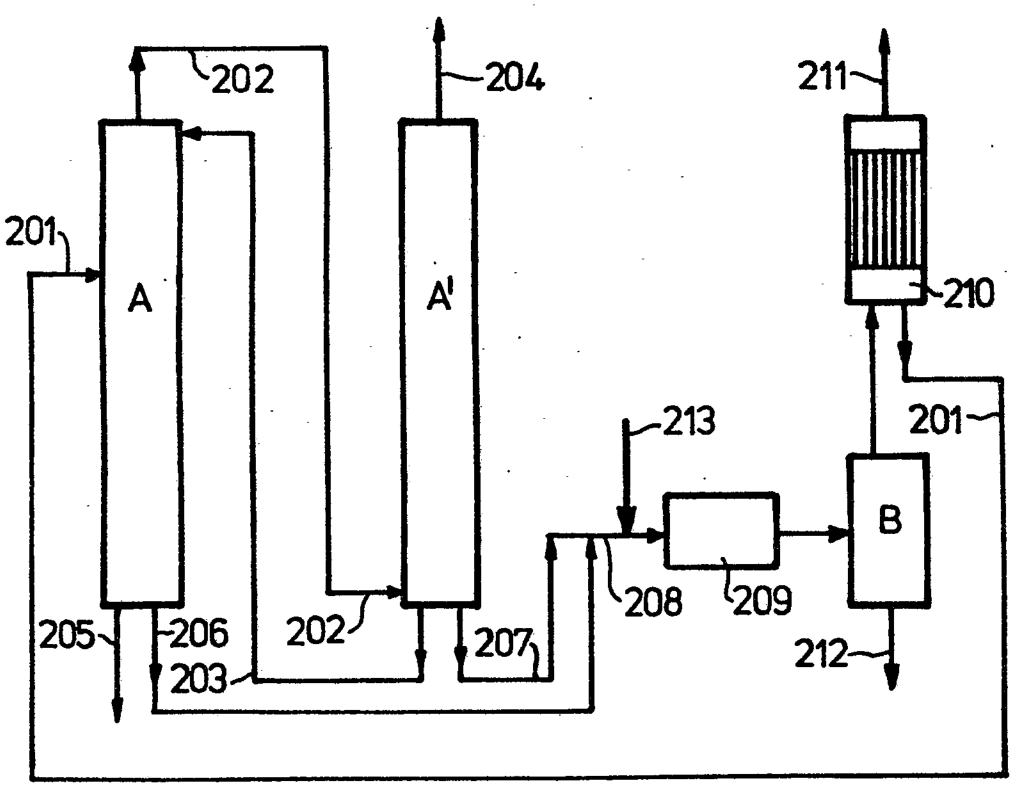
202
204
211
201
A
A'
210
213
201
208
209
B
205
206
202
207
203
212

FIG. 2

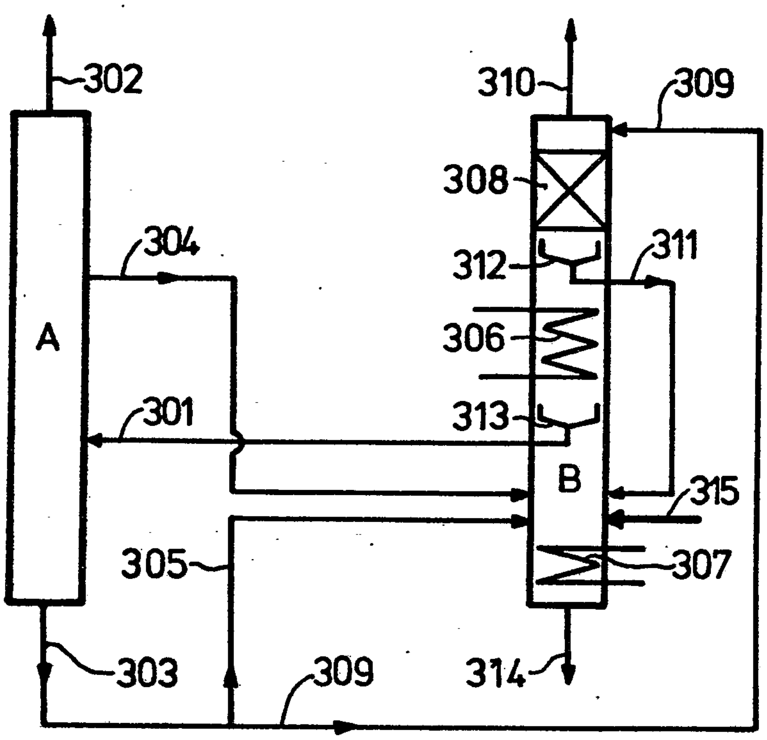
302
310
309
308
A
304
312
311
306
301
313
B
315
305
307
303
309
314

FIG. 3

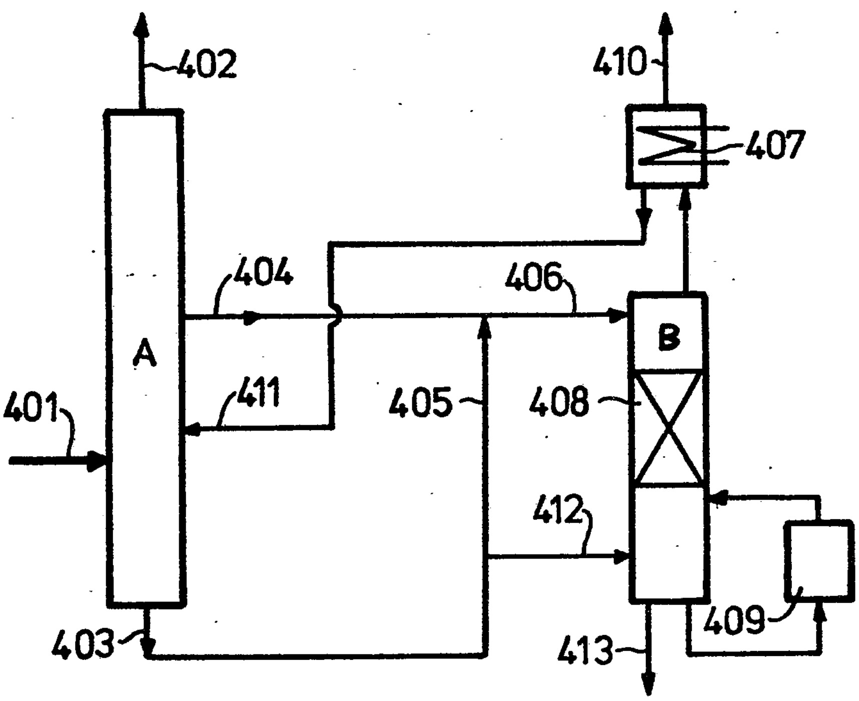
402
410
407
404
406
A
B
401
411
405
408
412
403
409
413

FIG. 4